# FASCICULE DE BREVET EUROPEEN

(11) **EP 0 698 381 B1**
(45) Date de publication et mention de la délivrance du brevet: **05.07.2000**
(21) Numéro de dépôt: 95401857.8
(22) Date de dépôt: 08.08.1995
(51) Int. Cl.: A61F 2/16

(54) **Implant intra-oculaire**
Intraokulares Linsenimplantat
Intraocular implant

(30) Priorité: 22.08.1994 FR 9410187
(43) Date de publication de la demande: 28.02.1996
(73) Titulaire: Crozafon, Philippe, F-06100 Nice (FR)
(72) Inventeur: Crozafon, Philippe, F-06100 Nice (FR)
(74) Mandataire: Bonnetat, Christian

(56) Documents cités:
- EP-A- 0 064 770
- EP-A- 0 180 887
- EP-A- 0 215 468
- EP-A- 0 337 390
- EP-A- 0 579 528
- FR-A- 2 651 117
- FR-A- 2 681 524
- DATABASE WPI Section PQ, Week 9044 Derwent Publications Ltd., London, GB; Class P, AN 90-333114 & SU-A-1 553 109 (EYE DISEASES RES. INST.)

## Description

La présente invention concerne un implant intra-oculaire destiné à être implanté, par une opération chirurgicale, dans le globe oculaire, et plus précisément dans le sac capsulaire de l'oeil.

Quoique non exclusivement, un tel implant intra-oculaire est plus particulièrement approprié à remplacer le cristallin naturel de l'oeil après une opération chirurgicale de la cataracte. On sait que la cataracte se caractérise par une opacité partielle ou totale du cristallin et nécessite souvent une intervention chirurgicale qui consiste à effectuer une ablation de ce cristallin devenu opaque, après section de la cornée et ouverture de la capsule. Une telle opération de la cataracte est généralement accompagnée par la mise en place, simultanée ou ultérieure, d'un implant intra-oculaire destiné à remplacer le cristallin opaque qui vient d'être ablaté.

On sait qu'un tel implant intra-oculaire comporte généralement, d'une part, une lentille centrale et, d'autre part, des moyens de fixation réalisés de préférence sous forme de bras élastiquement déformables et reliés par l'une de leurs extrémités à la périphérie externe de ladite lentille centrale. Ces bras sont destinés au positionnement et à la fixation de l'implant intra-oculaire de manière à rendre ce dernier solidaire de l'oeil.

Toutefois, par suite d'une mauvaise fixation au moyen desdits bras, il peut arriver que ledit implant intra-oculaire se déplace latéralement de manière à entraîner un décentrement de la lentille, ce qui peut empêcher une pénétration correcte des faisceaux lumineux dans l'oeil et provoquer en particulier des troubles de la vision.

Par ailleurs, la mise en place de l'implant intra-oculaire, qui représente un travail de grande précision, exige pour pouvoir être effectuée facilement, une forme adaptée de la lentille et des bras et ceci surtout lorsque l'incision de la cornée, par laquelle l'implant intra-oculaire est introduit dans le globe oculaire, est petite.

Il est évident que, lors d'une opération chirurgicale, on cherche à effectuer une incision la plus étroite possible, entre autres raisons pour atténuer d'éventuels problèmes post-opératoires dus à cette incision.

Par conséquent, la forme et les propriétés physiques, en particulier les propriétés mécaniques, des bras sont extrêmement importantes pour obtenir une mise en place facile et une fixation fiable et durable de l'implant intra-oculaire.

On notera que la forme à donner auxdits bras dépend essentiellement de la matière, dans laquelle est réalisée la lentille, et de la partie de l'oeil dans laquelle doit être implanté ledit implant intra-oculaire, d'une part, afin d'adapter les bras à la forme de cette partie et, d'autre part, en raison des contraintes que l'implant intra-oculaire est susceptible d'y subir et qu'il convient de maîtriser.

Ainsi, par exemple, un implant intra-oculaire mis en place dans le sac capsulaire d'un sujet opéré de la cataracte subit, en particulier, l'effet des contractions cicatricielles dudit sac capsulaire, lorsque ce dernier présente une fibrose cicatricielle post-opératoire.

De telles contractions cicatricielles peuvent engendrer un déplacement de l'implant intra-oculaire dans le sac capsulaire et provoquer le décentrement de la lentille, un tel décentrement pouvant entraîner une focalisation imparfaite des images sur la rétine, susceptible en particulier de créer des aberrations visuelles telles qu'une diplopie par exemple.

Par conséquent, en plus de permettre une mise en place facile, l'une des conditions essentielles à remplir par de tels implants intra-oculaires est de garantir une bonne fixation, et ceci quelles que soient les contraintes mécaniques qu'ils peuvent être amenés à subir.

On notera que le document EP-A-0 579 528 décrit un implant intra-oculaire comportant une lentille centrale et au moins deux bras de fixation élastiquement déformables et reliés par l'une de leurs extrémités à ladite lentille, chacun desdits bras présentant une direction générale radiale par rapport à ladite lentille, et comportant, à son extrémité libre opposée à ladite lentille, un bourrelet arqué convexe vers l'extérieur, la longueur curviligne dudit bourrelet arqué étant au moins égale au tiers du diamètre de la lentille et les bords desdits bras présentant une forme cintrée dans le plan de ladite lentille.

Ainsi, ledit implant intra-oculaire peut être fixé de façon relativement stable dans ledit sac capsulaire de forme arrondie, par l'intermédiaire desdits bourrelets qui, grâce à leur longueur importante et à leur forme arquée, permettent un contact important avec la paroi interne du sac capsulaire et diminuent les possibilités de déplacement latéral de l'implant intra-oculaire. Une telle fixation est renforcée par le fait que les parois du sac capsulaire en se cicatrisant l'une sur l'autre enveloppent et maintiennent lesdits bourrelets qui sont ainsi rendus solidaires du sac capsulaire.

De plus, en raison de la forme arquée des bourrelets, l'implant intra-oculaire peut être introduit facilement dans le sac capsulaire par rotation des bras sur la paroi interne dudit sac capsulaire.

En outre, grâce à leur direction générale radiale, lesdits bras élastiquement déformables sont susceptibles d'absorber d'éventuelles contraintes post-opératoires engendrées par la paroi du sac capsulaire, par exemple lors d'une fibrose cicatricielle, de manière à protéger la lentille centrale.

La présente invention a pour objet d'améliorer encore la fixation d'un tel implant. Elle concerne un implant intra-oculaire pouvant être implanté facilement et susceptible d'être fixé, en particulier dans le sac capsulaire de l'oeil, de façon fiable de manière à résister totalement à d'éventuelles réactions post-opératoires, telles qu'une fibrose cicatricielle par exemple.

A cette fin, selon l'invention, l'implant du type mentionné ci-dessus est caractérisé en ce que lesdits bras présentent une largeur sensiblement constante et comportent au moins un bourrelet supplémentaire agencé entre ladite lentille centrale et ledit bourrelet prévu à l'extrémité libre.

Ainsi, la fixation dans le sac capsulaire de l'implant intra-oculaire conforme à la présente invention est encore renforcée grâce au mode de solidarisation décrit précédemment, obtenu par la cicatrisation des parois du sac capsulaire l'une sur l'autre, en emprisonnant simultanément et partiellement les bras munis des bourrelets.

Lesdits bras peuvent présenter des ondes concentriques transversales par rapport à l'épaisseur des bras et concentriques par rapport au bourrelet correspondant.

Lesdites ondes concentriques apportent en outre une plus grande souplesse auxdits bras, en particulier dans le sens des contraintes de la paroi du sac capsulaire en contact avec lesdits bourrelets, ce qui permet d'absorber d'éventuelles contraintes mécaniques dudit sac capsulaire de manière à éviter un décentrement de la lentille susceptible d'être très dommageable comme rappelé précédemment.

En outre, avantageusement, lesdits bras présentent une largeur approximativement égale à la longueur de l'arc du bourrelet d'extrémité correspondant.

Pour faciliter la manipulation de l'implant intra-oculaire, on agence un nombre minimum de bras larges nécessaires au positionnement, à savoir deux bras, sur la lentille centrale de préférence de forme circulaire.

De plus, de façon avantageuse, ces deux bras sont identiques et sont agencés de façon symétrique, en plan, par rapport au centre de ladite lentille. Cette forme symétrique et simplifiée de l'implant intra-oculaire facilite également la manipulation et l'insertion dudit implant intra-oculaire.

Ladite lentille centrale peut évidemment présenter différentes formes dans le cadre de la présente invention, et en particulier un forme plan-convexe.

De préférence toutefois, la lentille centrale est réalisée sous forme d'un ménisque, qui est convexe du côté externe de l'implant intra-oculaire et concave du côté interne.

Une telle forme présente un double avantage. D'une part, ladite lentille se plie plus facilement qu'une lentille présentant une autre forme, par exemple une forme biconvexe, ce qui facilite la mise en place de l'implant intra-oculaire. D'autre part, la forme concave du côté interne permet de réduire la fibrose capsulaire, en raison d'un contact superficiel réduit de la lentille avec le sac capsulaire.

Les figures du dessin annexé feront bien comprendre comment l'invention peut être réalisée. Sur ces figures, des références identiques désignent des éléments semblables.

La figure 1 est une vue en plan d'un implant intra-oculaire à titre d'exemple.

La figure 2 est une coupe longitudinale de l'implant intra-oculaire, selon la ligne II-II de la figure 1.

La figure 3 est une vue en plan d'un implant intra-oculaire conforme à l'invention.

La figure 4 est une coupe longitudinale semblable à la coupe de la figure 2 d'un implant intra-oculaire réalisé selon un second mode de réalisation.

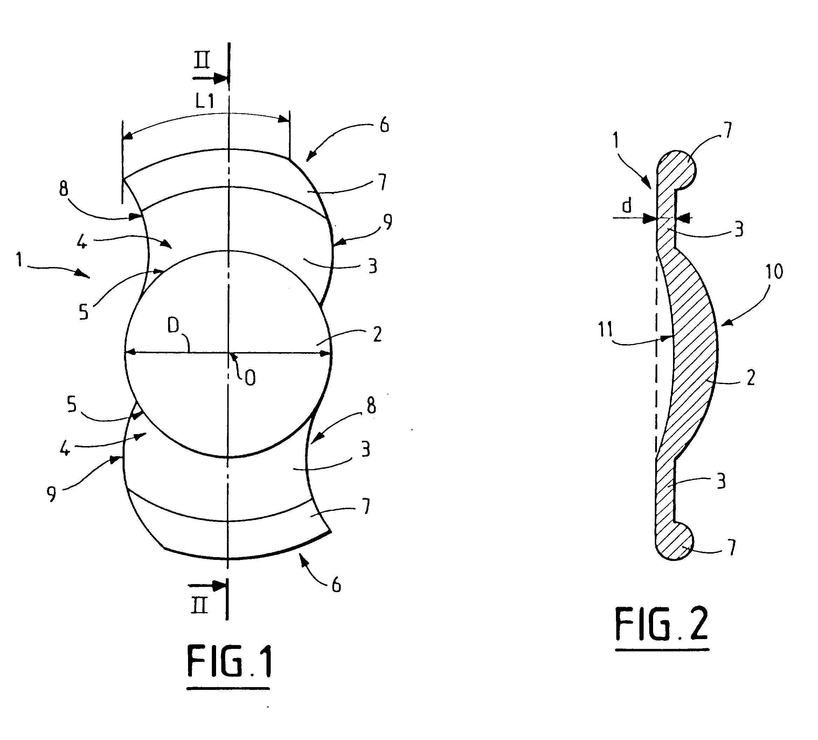

L'implant intra-oculaire 1, représenté sur les figures 1 et 2, peut par exemple être utilisé pour remplacer le cristallin naturel de l'oeil après une opération de la cataracte, ledit implant intra-oculaire 1 étant implanté à cet effet par exemple dans le sac capsulaire de l'oeil (non représenté).

Ledit implant intra-oculaire 1, réalisé de préférence en une seule pièce, soit en polyméthylméthacrylate, soit en un polymère thermoplastique spécial, comporte une lentille centrale circulaire 2, de centre O et de diamètre D, et des bras identiques 3, élastiquement déformables, reliés par l'une 4 de leurs extrémités à la périphérie externe 5 de ladite lentille centrale 2 et destinés à positionner et à fixer ledit implant intra-oculaire 1 dans le sac capsulaire de l'oeil. Lesdits bras 3 sont symétriques, en plan, par rapport audit centre O de la lentille 2. On notera, à titre d'exemple, que ledit implant intra-oculaire peut également être réalisé en silicone ou en acrylique.

Selon l'exemple des figures 1 et 2, chacun desdits bras 3 présente une direction générale radiale par rapport à ladite lentille 2, et comporte, à son extrémité libre 6 opposée à ladite lentille 2, un bourrelet arqué 7 convexe vers l'extérieur, la longueur curviligne L1 dudit bourrelet arqué 7 étant au moins égale au tiers du diamètre D de la lentille 2.

Dans l'exemple représenté, ladite longueur curviligne L1 est approximativement égale au diamètre D de la lentille 2.

De plus, l'arc de chacun desdits bourrelets 7 est centré sur la direction radiale du bras correspondant 3.

Comme on peut le voir sur la figure 2, lesdits bourrelets 7 font saillie vers l'extérieur de l'implant intra-oculaire 1, la partie de l'implant intra-oculaire destinée à être placée vers l'extérieur de l'oeil étant située à droite sur cette figure 2.

Lesdits bourrelets 7 présentent en outre une forme approximativement circulaire en section transversale, une telle forme n'étant toutefois pas exclusive, d'autres formes pouvant bien entendu être envisagées dans le cadre de la présente invention.

Par ailleurs, les bras 3 d'épaisseur d présentent d'une part des bords 8 et 9 cintrées de façon sensiblement identique, et d'autre part, une largeur sensiblement constante et approximativement égale à la longueur L1 de l'arc des bourrelets 7, tel que représenté sur la figure 1.

Grâce aux caractéristiques décrites ci-dessus, l'implant intra-oculaire 1 exemplaire présente en particulier les avantages suivants :
- l'extrémité libre 6 des bras 3 permet un contact important avec le sac capsulaire dans lequel doit être implanté l'implant intra-oculaire, en raison d'une part de la longueur curviligne L1 importante et d'autre part de la forme arquée des bourrelets 7 qui est adaptée à la forme arrondie dudit sac capsulaire ;
- ladite forme arquée des bourrelets 7 permet de plus une introduction chirurgicale facile de l'implant 1 par rotation de ladite extrémité libre 6 sur la paroi interne du sac capsulaire ; et
- lesdits bourrelets 7 permettent une solidarisation renforcée avec le sac capsulaire par fixation partielle des parois dudit sac capsulaire sur l'implant intra-oculaire.

En effet, après la mise en place de l'implant intra-oculaire dans le sac capsulaire, les parois dudit sac capsulaire s'affaissent de manière à entourer lesdits bourrelets et à venir au contact l'une de l'autre sur les côtés des bras. Ainsi, en se cicatrisant l'une sur l'autre au niveau de ces parties en contact, lesdites parois enveloppent les bourrelets des bras et les rendent solidaires du sac capsulaire, ce qui garantit une grande stabilité de l'implant intra-oculaire 1.

De plus, lesdits bras 3 ainsi réalisés de forme large et cintrée latéralement sont susceptibles d'absorber efficacement d'éventuelles contraintes post-opératoires engendrées par la paroi du sac capsulaire, par exemple lors d'une fibrose cicatricielle, de manière à protéger la lentille centrale 2, en évitant en particulier un décentrement de cette dernière.

Dans un mode de réalisation préféré de l'invention, le diamètre D de la lentille 2 et la longueur curviligne L1 sont approximativement égaux à 5,5 mm, l'épaisseur d à 0,5 mm et le diamètre du bourrelet 7 en section transversale est à peu près égal à 1 mm.

Comme on peut le voir sur la figure 2, ladite lentille centrale 2 est réalisée sous forme d'un ménisque qui est convexe du côté externe 10 et concave du côté interne 11.

Cette forme concave du côté interne 11 permet de réduire la fibrose capsulaire, en raison d'un contact superficiel réduit de la lentille 2 avec le sac capsulaire dudit côté interne 11, au contraire d'une forme plan-convexe ou bi-convexe également envisageable pour la lentille 2.

De plus, une lentille 2 ainsi réalisée se plie beaucoup plus facilement, ce qui facilite évidemment la mise en place de l'implant intra-oculaire 1.

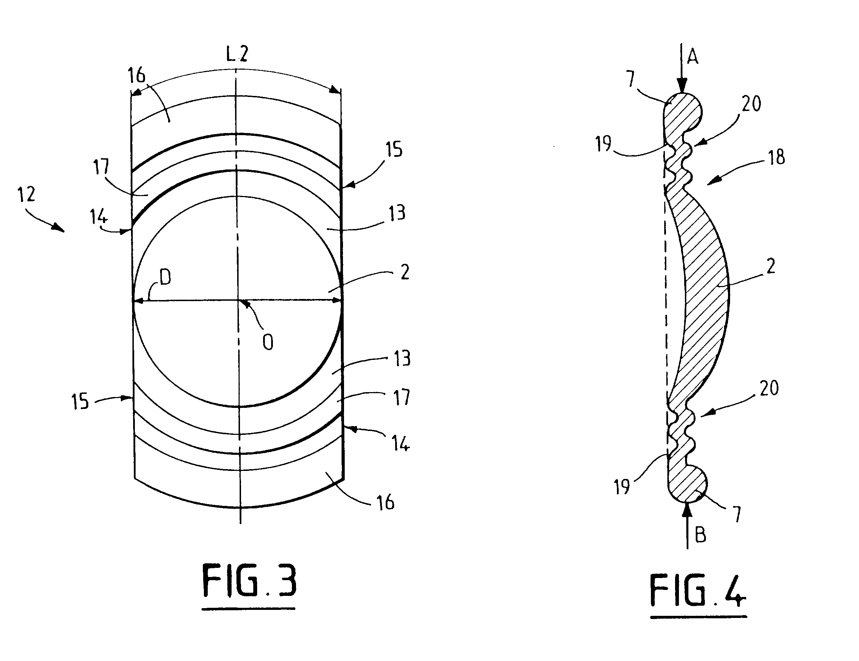

L'implant intra-oculaire 12, conforme à l'invention et représenté sur la figure 3, comporte deux bras identiques 13, symétriques par rapport au centre O de la lentille 2 et réalisés de façon rectiligne de sorte que, d'une part, leurs bords 14 et 15 sont parallèles entre eux, lesdits bords 14 et 15 étant espacés l'un de l'autre d'une distance approximativement égale au diamètre D de la lentille centrale 2, et d'autre part, le bord 14 de l'un desdits bras 13 correspond au prolongement du bord 15 de l'autre bras 13, et inversement.

En outre, selon l'invention, chacun desdits bras 13 est muni, en plus d'un bourrelet d'extrémité 16 de longueur curviligne L2 semblable au bourrelet 7 du bras 3, d'un second bourrelet 17 qui est agencé entre la lentille centrale 2 et ledit bourrelet 16 de manière à présenter une forme semblable et concentrique à la forme dudit bourrelet d'extrémité 16.

Bien entendu, il est possible de prévoir d'autres bourrelets supplémentaires sur lesdits bras 13.

Une telle réalisation selon l'invention permet de renforcer la fixation de l'implant intra-oculaire 12 dans le sac capsulaire, par suite de la cicatrisation des parois dudit sac capsulaire l'une sur l'autre, comme décrit précédemment.

En effet, on obtient, grâce auxdits bourrelets supplémentaires 17, de nouveaux éléments d'ancrage pour lesdites parois du sac capsulaire, permettant d'augmenter la stabilité par rapport audit sac capsulaire.

Dans une variante de l'invention, tel que représenté sur la figure 4, l'implant intra-oculaire 18 comporte des bras 19 présentant en section une configuration en forme d'ondes 20, lesdites ondes 20 étant formées entre les bourrelets 7 agencés à l'extrémité libre des bras 19 et la lentille centrale 2. Pour le reste, ledit implant intra-oculaire 1 est identique à l'implant intra-oculaire 3, comme on peut le voir en comparant les figures 2 et 4.

Lesdites ondes 20 sont concentriques et présentent, en vue de face non représentée, une forme arrondie correspondant à la forme arrondie des bourrelets 7.

Grâce auxdites ondes concentriques 20, les bras 19 présentent une plus grande souplesse en particulier dans des directions indiquées par des flèches A et B et correspondant aux directions d'éventuelles contraintes susceptibles d'être exercées sur l'implant intra-oculaire 18 par la paroi du sac capsulaire qui se trouve en contact des bourrelets 7 après l'implantation. Cette souplesse permet donc auxdits bras 19 d'absorber de telles contraintes mécaniques dudit sac capsulaire et ainsi d'éviter un décentrement de la lentille centrale, un tel décentrement étant susceptible de provoquer des anomalies visuelles.

## Revendications

1. Implant intra-oculaire (12, 18) comportant une lentille centrale (2) et au moins deux bras de fixation (13, 19) élastiquement déformables et reliés par l'une de leurs extrémités à ladite lentille (2), chacun desdits bras (13, 19) présentant une direction générale radiale par rapport à ladite lentille (2), et comportant, à son extrémité libre opposée à ladite lentille (2), un bourrelet arqué (7, 16) convexe vers l'extérieur, la longueur curviligne (L1, L2) dudit bourrelet arqué (7, 16) étant au moins égale au tiers du diamètre (D) de la lentille (2) et les bords (8, 9) desdits bras présentant une forme cintrée dans le plan de ladite lentille (2),
caractérisé en ce que lesdits bras (13) présentent une largeur sensiblement constante et comportent au moins un bourrelet supplémentaire (17) agencé entre ladite lentille centrale (2) et ledit bourrelet (7, 16) prévu à l'extrémité libre.

2. Implant intra-oculaire selon la revendication 1,
caractérisé en ce que lesdits bras (19) présentent des ondes concentriques (20) transversales par rapport à l'épaisseur des bras (19) et concentriques par rapport au bourrelet correspondant (7).

3. Implant intra-oculaire selon l'une des revendications 1 ou 2, caractérisé en ce que la largeur desdits bras est approximativement égale à la longueur de l'arc du bourrelet d'extrémité correspondant.

4. Implant intra-oculaire selon l'une des revendications 1 à 3, à deux bras (3, 13, 19), dont la lentille centrale (2) est de forme circulaire, caractérisé en ce que les deux bras (13, 19) sont identiques et sont agencés de façon symétrique, en plan, par rapport au centre (O) de la lentille (2).

5. Implant intra-oculaire selon l'une quelconque des revendications 1 à 4,
caractérisé en ce que la lentille centrale (2) est réalisée sous forme d'un ménisque, qui est convexe du côté externe (10) de l'implant intra-oculaire et concave du côté interne (11).

## Patentansprüche

1. Intraokulares Implantat (12, 18), aufweisend eine zentrale Linse (2), und mindestens zwei elastisch deformierbare Befestigungsarme (13, 19), die durch eines ihrer Enden mit der Linse (2) verbunden sind, wobei jeder der Arme (13, 19) eine radiale Hauptrichtung zur Linse (2) zeigt und an seinem freien Ende gegenüber der Linse (2) einen konvex nach außen gebogenen Wulst (7, 16) aufweist, wobei die Länge der gebogenen Linie (L1, L2) des gebogenen Wulstes (7, 16) mindestens gleich einem Drittel des Durchmessers (D) der Linse (2) ist und die Ränder (8, 9) der Arme eine gewölbte Form in der Ebene der Linse (2) aufweisen,
dadurch gekennzeichnet, daß die Arme (13) eine im wesentlichen konstante Breite zeigen und mindestens einen zusätzlichen Wulst (17) aufweisen, der zwischen der zentralen Linse (2) und dem am freien Ende vorgesehenen Wulst (7, 16) angeordnet ist.

2. Intraokulares Implantat nach Anspruch 1, dadurch gekennzeichnet, daß die Arme (19) konzentrische Wellen (20) quer zur Dicke der Arme (19) und konzentrisch zum entsprechenden Wulst (7) zeigen.

3. Intraokulares Implantat nach einem der Ansprüche 1 oder 2, dadurch gekennzeichnet, daß die Breite der Arme etwa gleich der Länge des Bogens des Wulstes des entsprechenden Endes ist.

4. Intraokulares Implantat nach einem der Ansprüche 1 bis 3, mit zwei Armen (3, 13, 19), wobei die zentrale Linse (2) kreisförmig ist, dadurch gekennzeichnet, daß die zwei Arme (13, 19) identisch sind und spiegelsymmetrisch zum Zentrum (O) der Linse (2) angeordnet sind.

5. Intraokulares Implantat gemäß irgendeinem der Ansprüche 1 bis 4, dadurch gekennzeichnet, daß die zentrale Linse (2) in Form eines Meniskus realisiert ist, der konvex zur Außenseite (10) und konkav zur Innenseite (11) des intraokularen Implantats ist.

## Claims

1. Intraocular implant (12,18) comprising a central lens (2) and at least two elastically deformable securing arms (13, 19) which are connected via one of their ends to said lens (2), each of said arms (13,19) exhibiting a general direction which is radial with respect to said lens (2), and comprising, at its free end opposite said lens (2), an arched bead (7,16) convex toward the outside, the curvilinear length (L1, L2) of said arched bead (7,16) being at least equal to a third of the diameter (D) of the lens (2) and the edges (8,9) of said arms having a shape curved in the plane of said lens (2),
characterized in that said arms (13) have a substantially constant width and comprise at least one supplementary bead (17) disposed between said central lens (2) and said bead (7, 16) provided at the free end.

2. Intraocular implant according to claim 1,
characterized in that said arms (19) have concentric undulations (20) transverse with respect to the thickness of the arms (19) and concentric with respect to the corresponding bead (7).

3. Intraocular implant according to one of claims 1 or 2,
characterized in that the width of said arms is substantially equal to the length of the arc of the corresponding end bead.

4. Intraocular implant according to one of claims 1 to 3, with two arms (3, 13, 19) of which the central lens (2) is of circular shape,
characterized in that the two arms (13, 19) are identical and are arranged in a symmetrical manner, in one plane, with respect to the center (0) of the lens (2).

5. Intraocular implant according to any one of claims 1 to 4,
characterized in that the central lens (2) is made in the shape of a meniscus which is convex on the outer side (10) of the intraocular implant and concave on the inner side (11).
